# FASCICULE DE BREVET EUROPEEN

(11) **EP 2 056 846 B1**
(45) Date de publication et mention de la délivrance du brevet: **03.07.2013**
(21) Numéro de dépôt: 07823749.2
(22) Date de dépôt: 28.08.2007
(51) Int. Cl.: A61K 35/54, A61K 36/58, A61K 36/82, A61K 31/7012, A61K 31/728, A61K 33/00, A61K 38/40

(54) **SUBSTITUT SALIVAIRE**
SPEICHELERSATZ
SALIVARY SUBSTITUTE

(30) Priorité: 01.09.2006 FR 0653549
(43) Date de publication de la demande: 13.05.2009
(73) Titulaire: Unither Developpement, 80080 Amiens (FR)
(72) Inventeur: DEYMES, Jean, F-33000 Bordeaux (FR); PEROVITCH, Philippe, F-33680 Le Temple (FR)
(74) Mandataire: Riege, Christian
(86) Numéro de dépôt international: PCT/FR2007/051850
(87) Numéro de publication internationale: WO 2008/025926

(56) Documents cités:
- WO-A-99/04804
- US-A1- 2005 226 822
- WOLINSKY L E ET AL: "The inhibiting effect of aqueous Azadirachta indica (neem) extract upon bacterial properties influencing in vitro plaque formation" JOURNAL OF DENTAL RESEARCH, vol. 75, no. 2, 1996, pages 816-822, XP002426645 ISSN: 0022-0345

## Description

La présente invention concerne un substitut de la salive naturelle élaboré à partir de blanc d'oeuf.

L'invention se rapporte également à ses utilisations et à son conditionnement. L'insuffisance salivaire ou xérostomie est une pathologie très répandue qui conduit à des effets indésirables importants au niveau de la sphère bucco pharyngée. Ses origines sont multiples : affections immunitaires, effets secondaires de nombreux médicaments, âge, ménopause ou encore traitements de cancers buccopharyngés par radiothérapie. Elle entraîne une sécheresse buccale qui peut avoir des conséquences graves allant des troubles de la phonation à l'anorexie, avec des effets psycho-dépressifs.

Ces problèmes extrêmement gênants provoqués par les xérostomies sont bien connus mais il n'existe actuellement aucun traitement satisfaisant permettant de rééquilibrer l'atmosphère buccale.

Une solution pour lutter contre la sécheresse buccale engendrée par une insuffisance salivaire est d'administrer une salive reconstituée.

La salive est un liquide biologique qui humidifie les muqueuses de la bouche et du pharynx, facilite la phonation, la mastication et la déglutition. Elle possède également un rôle antiseptique et un rôle de protection de l'oesophage.

La salive est un milieu très spécifique et composite, complexe, constitué en grande partie d'eau. Ce milieu est à la fois reflet de la composition plasmatique et porteur de substances synthétisées par les glandes salivaires exocrines en particulier des protéines de haut poids moléculaire dont les mucines, les globulines, des glucoprotéines, des enzymes, des minéraux, des composés intervenant dans la protection anti-infectieuse. Sa composition équilibrée et multifonctionnelle est difficile à reconstituer fidèlement et de manière stable compte tenu des interactions délicates et subtiles intervenant sans cesse entre ses différents composants.

Il existe un certain nombre de produits qui tentent de remplacer l'effet de la salive.

On connaît par exemple un produit à base de sels minéraux, de sorbitol et d'eau. On peut citer également un gel qui contient principalement des substances polymériques d'origine extractive ou synthétique, des polyols et des enzymes dont la stabilité est incertaine dans cette ambiance.

D'autres préparations se présentent sous forme de principes actifs synthétiques, dont notamment une à base d'oxyde de Polyéthylène.

Il existe aussi un produit constitué de Triesters de Glycérol oxydé (Aequasyal®) qui, appliqué sur des muqueuses déjà asséchées et inflammatoires, forme un film lipidique peu susceptible de constituer un substrat actif et propice aux échanges biologiques.

Enfin, des mucines extraites d'estomac de porc on été utilisées associées à des minéraux et des Polyols pour tenter de reproduire l'effet des mucines salivaires. Tous ces produits sont extrêmement éloignés de la complexité rhéologique, de l'effet muco-protecteur et de l'activité biologique de la salive naturelle. Ils présentent une composition et des comportements différents, ce qui ne leur permet pas de fournir une fonctionnalité équivalente aussi complexe même approchée, ni de participer à l'homéostasie buccale.

On connaît aussi des demandes de brevet et brevets traitant de ce sujet.

Ainsi, la demande de brevet américaine US 2005/0226822 décrit un substitut de la salive humaine comprenant de l'ovomucine issue de blanc d'oeuf. Cette demande prévoit exclusivement cette utilisation d'un composé du blanc d'oeuf excluant alors les autres composants du blanc d'oeuf complet.

La demande internationale de brevet WO 99/04804 se rapporte à l'utilisation de sécrétions immunes, à partir de lait ou d'oeuf, obtenues à partir d'animaux auxquels on a administré des éléments pathogènes. Ce document vise des applications incluant tant la xérostomie que les caries dentaires ou les inflammations buccales. L'obtention de telles compositions est complexe et requiert la mise en place d' une chaîne incluant les animaux eux-mêmes.

Il subsiste donc un besoin pour une salive reconstituée avec une composition et des qualités analogues à la salive naturelle, avec des moyens de réalisation et de production industriels.

C'est pourquoi l'objectif de la présente invention est de proposer un substitut salivaire à base d'un milieu biologique facilement disponible qui mime la composition et les équilibres complexes de la salive.

A cet effet l'invention vise un substitut salivaire obtenu à partir de blanc d'oeuf. Ce substitut présente des propriétés chimiques, rhéologiques et physiologiques analogues à celles de la salive humaine naturelle.

Avantageusement le blanc d'oeuf est un composé protéique naturel rassemblant la quasi-totalité des composants de la salive, dans un équilibre équivalent. Il possède également un pouvoir couvrant similaire et une viscosité analogue, permettant la coexistence des différents composants entre eux.

Selon un autre avantage de l'invention, le blanc d'oeuf est un milieu naturellement aseptique dont la stabilité de composition est bien connue.

L'invention est maintenant décrite en détail suivant des modes de réalisation particuliers, non limitatifs, avec une illustration par un conditionnement dans un contenant particulier.

Le substitut salivaire selon l'invention comprend au moins du blanc d'oeuf.

Le blanc d'oeuf est composé d'un ensemble d'éléments d'origine organique, similaires à ceux de la salive humaine physiologique, ce qui le rend particulièrement adapté à la réalisation d'un substitut salivaire proche de la salive naturelle.

Le blanc d'oeuf est un produit stable.

La salive naturelle est constituée de protéines de haut poids moléculaire qui lui fournissent sa viscosité, son pouvoir couvrant des muqueuses et des dents en même temps qu'elles assurent les échanges et fixations de minéraux sur l'émail dentaire. Ces protéines sont principalement des mucines et des Immuno Globulines A, glycoprotéines acides.

Les protéines du blanc d'oeuf sont analogues en terme de nature, de poids moléculaire et de structure, aux protéines constitutives de la salive. On peut citer en particulier :
- l'ovalbumine, qui représente 54% des protéines à très grosse structure moléculaire,
- l'ovotransferrine présente à 12-13%, qui fixe les atomes de fer, bloquant la reproduction bactérienne,
- l'ovomucoïde, présente à 11%, inhibiteur des protéases,
- des ovoglobulines G2, G3, ovoflavoprotéines, ovoinhibiteurs, de poids moléculaire de 50 KDa, présentes à environ 5%,
- l'ovomucine présente à 3,5%, mucine à très gros poids moléculaire (entre 210 et 720 KDa) qui interagit avec l'ovalbumine, l'ovotransferrine et le lysozyme, et
- l'ovomacroglobuline de poids moléculaire très élevé (entre 760 et 900 KDa) pour 0,5%.

Le blanc d'oeuf contient également une enzyme particulière de la salive, le lysozyme.

En ce qui concerne les apports minéraux du blanc d'oeuf, en particulier le rapport Na/K, ils sont tout à fait adaptés aux besoins d'une salive naturelle. De plus, les apports de calcium, de phosphore, de magnésium, de soufre et de chlore répondent aux exigences de minéralisation de l'espace bucco-dentaire.

Selon un mode de réalisation de l'invention, il est possible d'ajouter au blanc d'oeuf des substances qui vont accroître sa compétence d'humidification, de protection biologique des muqueuses et des dents, d'échanges physico-chimiques, d'adoucissement des tissus inflammatoires et de rééquilibrage du pH acide des tissus vers un pH neutre.

La composition comprend des substances permettant de régler la viscosité du substitut salivaire. En effet, la salive naturelle mousse et se charge spontanément d'air dans la cavité buccale formant une phase mixte d'air et d'eau liés par des phénomènes de tensio activité.

Ainsi le substitut salivaire à base de blanc d'oeuf selon l'invention comprend au moins une substance permettant le réglage de la viscosité entre 1 et 5 poises, pour approcher celle d'une bouche saine comprise entre 1,25 et 1,35 poises, et produisant une tension superficielle entre 16 et 22 dyne/cm⁻¹. Cette substance est choisie parmi :
- dérivés cellulosiques :
   ✔ carboxy - méthyl cellulose sodique,
   ✔ hydroxy-éthyl cellulose
   ✔ hydroxy-propyl cellulose
   ✔ hydroxy-propyl méthyl cellulose ou hypromelllose
   ✔ carboxy-méthyl cellulose
- gommes :
   ✔ guar
   ✔ xanthane
   ✔ gomme arabique
- polymères non cellulosiques :
   ✔ acide alginique et dérivés,
   ✔ polymères carboxy-vinyliques
   ✔ carbomères
   ✔ macrogols
   ✔ polyéthylène glycol
   ✔ gélatine
   ✔ povidone
   ✔ pectines

On peut aussi choisir d'autres structures polymériques comme l'acide hyaluronique ou des polyols tels que sorbitol, mannitol.

Pour répondre aux exigences d'un substitut salivaire adapté, il est également important d'ajuster le pH en vue de restituer à l'atmosphère buccale un pH neutre, voire idéalement proche de 7 dans ces bouches pathologiquement acides. En effet, le pH légèrement alcalin du substitut, préférentiellement compris entre 7,5 et 9, permet de protéger la stabilité de certaines substances d'origine naturelle comme le lysozyme et permet de tamponner le pH buccal à un niveau physiologique.

Ainsi le substitut salivaire à base de blanc d'oeuf selon l'invention peut aussi comprendre au moins une substance permettant l'ajustement du pH entre 7,5 et 9, préférentiellement par constitution d'un effet tampon à base de bicarbonate de sodium et de carbonate de calcium ou d'orthophosphate de calcium.

A ces éléments, on peut éventuellement associer des compléments spécifiques destinés notamment à compenser les déficits particuliers liés aux xérostomies, à savoir notamment :
- de l'acide sialique ou acide N-Acetyl-Neuraminique, déjà présent dans la majorité des protéines du blanc d'oeuf,
- de l'acide hyaluronique, molécule largement répandue dans tous les tissus de l'organisme, pouvant jouer un rôle de capteur d'hydratation et de mucoprotecteur pour adoucir et protéger l'atmosphère buccale,
- de la lactoferrine, pour accroître la capacité de défense du milieu contre les bactéries, virus et agents mycosiques,
- des polyols, notamment du sorbitol ou du mannitol pour leur aspect rétenteur d'humidité,
- du fluorure de sodium ou de calcium qui inhibe par simple contact la formation des acides cariogènes dans ces bouches xérostomiques, notamment dosés à 0,5/1 mg/l,
- des extraits naturels, en particulier un extrait de Neem qui agit sur les glucans et bactéries constitutives de la plaque dentaire, et/ou
- un extrait de thé vert, riche en fluor réducteur de caries et en polyphénols réducteurs d'halitose, qui participe également à prévenir les gingivites et la prolifération bactérienne.

On peut également ajouter du lysozyme, bien que le blanc d'oeuf en contienne déjà en quantité suffisante, pour compenser d'éventuelles pertes.

Le substitut salivaire selon l'invention a une constitution analogue à celle d'une salive naturelle physiologique. Il peut être avantageusement utilisé pour fournir aux bouches asséchées des éléments indispensables à un apaisement et un rétablissement de l'homéostasie.

La salive reconstituée selon l'invention est donc utile pour compenser et réparer une insuffisance salivaire et/ou maintenir une humidité résiduelle plus importante dans les bouches déficitaires en salive.

Selon un aspect de l'invention, le substitut salivaire peut être présenté sous forme liquide ou gel.

Un exemple de formulation peut être donné à titre indicatif :
- poudre de blanc d'oeuf: 0,5 g
- sorbitol : 1 g
- bicarbonate de sodium : 0,5 g
- carbonate de calcium : 0,5 g
- fluorure de sodium : 0,025 mg
- Natrosol® 250 HX polymère : 0,5 g
- eau : 100 ml
- arômes : qsp pour neutralisation du goût.

Préférentiellement, le substitut salivaire est conditionné sous forme d'unidoses de 2 à 5 ml, susceptibles de fournir le volume adéquat de garnissage salivaire d'une bouche xérostomique.

Pour la protection de la stabilité de la composition et l'imperméabilité à l'oxygène et aux rayonnements mais aussi pour le confort d'utilisation par le patient, pour un transport aisé, on peut préférentiellement recourir aux emballages "stick". Ces emballages, sous forme d'étuis étanches spécifiques, imperméables à la lumière et à l'oxygène, sont réalisés à partir d'une enveloppe métalloplastique souple. Ils assurent la stabilité physico-chimique dudit substitut.

De façon avantageuse, ce conditionnement est facile à transporter et permet une utilisation aisée du substitut salivaire à tout moment de la journée.

Sur le plan de l'action, on comprend que l'utilisation d'un substitut salivaire analogue à la salive naturelle est très efficace et procure un confort pour les personnes atteintes de xérostomies.

L'effet pour une dose peut se prolonger pendant une heure environ.

Ainsi l'utilisation d'une dose de substitut salivaire selon l'invention permet aux personnes souffrant de xérostomies, de parler facilement ou de prendre des repas dans de bonnes conditions par exemple.

## Revendications

1. Substitut salivaire présentant des propriétés chimiques, rhéologiques et physiologiques analogues à celles de la salive humaine naturelle, comprenant du blanc d'oeuf et au moins une substance permettant de régler la viscosité entre 1 et 5 poises et la capacité moussante, dans lequel ladite au moins une substance permettant de régler la viscosité et la capacité moussante est un polymère de poids moléculaire élevé, et/ou de l'acide hyaluronique et/ou un polyol, ledit polymère de poids moléculaire élevé étant choisi dans le groupe constitué de : carboxy-méthyl cellulose sodique, hydroxy-éthyl cellulose, hydroxy-propyl cellulose, hydroxy-propyl méthyl cellulose, carboxy-méthyl cellulose, gomme de guar, gomme de xanthane, gomme arabique, acide alginique et dérivés, polymères carboxy-vinyliques, carbomères, macrogols, polyéthylène glycol, gélatine, povidone et pectines, et ledit polyol étant du sorbitol ou du mannitol.

2. Substitut salivaire selon la revendication 1, comprenant en outre au moins une substance permettant d'ajuster le pH du substitut entre 7,5 et 9.

3. Substitut salivaire selon la revendication 2, dans lequel la substance permettant d'ajuster le pH entre 7,5 et 9 et de constituer un effet tampon est à base de bicarbonates et de carbonates.

4. Substitut salivaire selon l'une quelconque des précédentes revendications, comprenant du blanc d'oeuf et du lysozyme, de l'acide sialique, de l'acide hyaluronique, de la lactoferrine, un extrait de Neem, un extrait de thé vert, des polyols et/ou des fluorures de sodium ou de calcium.

5. Utilisation du substitut salivaire selon l'une quelconque des précédentes revendications pour compenser une insuffisance salivaire.

6. Utilisation du substitut salivaire selon l'une des revendications 1 à 4, pour maintenir une humidité résiduelle importante dans les bouches déficitaires en salive.

7. Contenant incluant le substitut salivaire selon l'une des revendications 1 à 4, **caractérisé en ce qu'**il se présente sous forme d'un étui étanche unidose de 2 à 5 ml, imperméable à la lumière et à l'oxygène, réalisé à partir d'une enveloppe métalloplastique souple.

## Patentansprüche

1. Speichelersatz, der chemische, rheologische und physiologische Eigenschaften analog zu denjenigen von natürlichem menschlichem Speichel hat, aufweisend Eiweiß und mindestens eine Substanz, die eine Einstellung der Viskosität auf zwischen 1 und 5 Poise sowie des Schaumbildungsvermögens ermöglicht, wobei es die mindestens eine Substanz, die eine Einstellung der Viskosität und des Schaumbildungsvermögens ermöglicht, ein Polymer mit hohem Molekulargewicht und/oder Hyaluronsäure und/oder ein Polyol ist, wobei das Polymer mit hohem Molekulargewicht aus der aus Folgendem bestehenden Gruppe ausgewählt ist: Natrium-Carboxymethylcellulose, Hydroxyethylcellulose, Hydroxypropylcellulose, Hydroxypropylmethylcellulose, Carboxymethylcellulose, Guargummi, Xanthangummi, Arabisches Gummi, Alginsäure und Derivate, Carboxyvinylpolymere, Carbomere, Macrogole, Polyethylenglycol, Gelatine, Povidon und Pektine, wobei das Polyol Sorbit oder Mannitol ist.

2. Speichelersatz nach Anspruch 1, des Weiteren aufweisend mindestens eine Substanz, die die Einstellung des pH-Wertes des Ersatzes auf zwischen 7,5 und 9 ermöglicht.

3. Speichelersatz nach Anspruch 2, wobei die Substanz, die eine Einstellung des pH-Wertes auf zwischen 7,5 und 9 und die Bildung einer Pufferwirkung ermöglicht, auf Bicarbonaten und Carbonaten basiert.

4. Speichelersatz nach einem der vorhergehenden Ansprüche, aufweisend Eiweiß und Lysozym, Sialsäure, Hyaluronsäure, Lactoferrin, ein Neem-Extrakt, ein Extrakt aus grünem Tee, Polyole und/oder Natrium- oder Calciumfluoride.

5. Verwendung des Speichelersatzes nach einem der vorhergehenden Ansprüche zum Ausgleichen einer Speichelinsuffizienz.

6. Verwendung des Speichelersatzes nach einem der Ansprüche 1 bis 4 zum Erhalten einer erheblichen Restfeuchtigkeit im Mund mit einem Speicheldefizit.

7. Behälter, der den Speichelersatz nach einem der Ansprüche 1 bis 4 enthält, **dadurch gekennzeichnet, dass** er in Form einer dichten Kapsel mit einer einzigen Dosis von 2 bis 5 ml vorliegt, die gegenüber Licht und Sauerstoff undurchlässig ist und die aus einem flexiblen Metall-Kunststoffmantel hergestellt ist.

## Claims

1. Salivary substitute having chemical, rheological and physiological properties that are analogous to those of natural human saliva, comprising egg white and at least one substance that makes it possible to adjust the viscosité between 1 and 5 poise and the foaming capacity, wherein said at least one substance that makes it possible to adjust the viscosité and the foaming capacity is a polymer of high molecular weight and/or hyaluronic acid and/or a polyol, said polymer of high molecular weight being chosen in the group consisting of: sodium carboxymethyl cellulose, hydroxyethyl cellulose, hydroxypropyl cellulose, hydroxypropyl methyl cellulose, carboxymethyl cellulose, guar gum, xanthan gum, gum Arabic, alginic acid and derivatives, carboxy-vinyl polymers, carbomers, macrogols, polyethylene glycol, gelatin, povidone and pectins, and said polyol being sorbitol or mannitol.

2. Salivary substitute according to claim 1, further comprising at least one substance that makes it possible to adjust the pH of the substitute between 7.5 and 9.

3. Salivary substitute according to claim 2,
wherein the substance that makes it possible to adjust the pH between 7.5 and 9 and to constitute a buffer effect is based on bicarbonates and carbonates.

4. Salivary substitute according to any of the preceding claims, wherein it comprises egg white and lysozyme, sialic acrid, hyaluronic acid, lactoferrin, a Neem extract, a green tea extract, polyols, and/or sodium or calcium fluorides.

5. Use of the salivary substitute according to any of the preceding claims for compensating for a salivary deficiency.

6. Use of the salivary substitute according to one of claims 1 to 4 for maintaining a significant level of residual moisture in saliva-deficient mouths.

7. Container that includes the salivary substitute according to one of claims 1 to 4, wherein it comes in the form of an airtight, single-dose case of 2 to 5 ml, impermeable to light and to oxygen, produced from a flexible metalloplastic jacket.
